# EUROPEAN PATENT SPECIFICATION

(11) **EP 1 426 765 B1**
(45) Date of publication and mention of the grant of the patent: **29.03.2006**
(21) Application number: 03026551.6
(22) Date of filing: 18.11.2003
(51) Int. Cl.: G01N 33/569, G01N 33/68, C07K 14/315

(54) **Method for examining the risk of dental caries**
Verfahren zur Bestimmung des Risikos von Dentalkaries
Procédé pour évaluer le risque de caries dentaires

(30) Priority: 04.12.2002 JP 2002352466
(43) Date of publication of application: 09.06.2004
(73) Proprietor: GC Corporation, Itabashi-ku, Tokyo 174 (JP)
(72) Inventor: Senpuku, Hidenobu, Yokohama-shi Kanagawa-ken (JP); Masuzawa, Yumiko, GC Corporation, Tokyo (JP)
(74) Representative: Müller-Boré & Partner Patentanwälte

(56) References cited:
- OZAWA Y ET AL: "HLA class II alleles and salivary numbers of mutans streptococci and lactobacilli among young adults in Japan." ORAL MICROBIOLOGY AND IMMUNOLOGY. DENMARK DEC 2001, vol. 16, no. 6, December 2001 (2001-12), pages 353-357, XP002272025 ISSN: 0902-0055
- ACTON RONALD T ET AL: "Associations of MHC genes with levels of caries-inducing organisms and caries severity in African-American women" HUMAN IMMUNOLOGY, vol. 60, no. 10, October 1999 (1999-10), pages 984-989, XP002272026 ISSN: 0198-8859
- WALLENGREN M L L ET AL: "HLA-DR4 and salivary immunoglobulin A reactions to oral streptococci" ORAL MICROBIOLOGY AND IMMUNOLOGY, vol. 16, no. 1, February 2001 (2001-02), pages 45-53, XP002272109 ISSN: 0902-0055
- SENPUKU H ET AL: "Identification of Streptococcus mutans PAc peptide motif binding with human MHC class II molecules (DRBI*0802, *1101, *1401 and *1405)" IMMUNOLOGY, vol. 95, no. 3, November 1998 (1998-11), pages 322-330, XP008028150 ISSN: 0019-2805
- SENPUKU H ET AL: "Inhibitory effects of MoAbs against a surface protein antigen in real-time adherence in vitro and recolonization in vivo of Streptococcus mutans" SCANDINAVIAN JOURNAL OF IMMUNOLOGY, vol. 54, no. 1-2, July 2001 (2001-07), pages 109-116, XP002272027 ISSN: 0300-9475

## Description

The present invention relates to a method for examining a caries risk by using a human gene diagnosis.

It has been known that there are close relation between existence of mutans streptococci in a human oral cavity and generation of caries, and many researches have been reported thereof, for example, Anders Thylstrup and Ole Fejerskov, "Textbook of clinical caliology" (Denmark), Munksgaard Corporation, 1996, 2nd edition, p.405. The mutans streptococci is the general term of Streptococcus mutans and Streptococcus sobrinus (hereinafter, they are referred to as "S. mutans" and "S. sobrinus" respectively) as strains existing in human saliva.

Since the new caries may be generated further in the future if there is much quantity of the mutans streptococci existing in the human saliva, many trials for easily determining the quantity of the mutans streptococci in the saliva have been carried out. For example, there are the methods proposed in Japanese Patent Laid-Open No. 02177898 and Japanese Patent Laid-Open No. 10036400, in which the mutans streptococci quantity is determined by applying monoclonal antibody specifically reacted with the S. mutans, and the method proposed in United States Patent No. 5,374,538 , in which the quantity of bacterial cells themselves grown in a simplified culturing kit is determined by visual observation. However, in order to examine a caries risk by measuring the quantity of the mutans streptococci existing in saliva, there are problems that the mutans streptococci should actually exist in the oral cavity and caries has been already generated in many cases at the time of judging that there is a high caries risk.

On the other hand, it has been known that an increasing process of the quantity of the mutans streptococci is varied with persons, and if the difference of said increasing processes can be examined beforehand, the caries risk can be known irrespective of the actual increasing or quantity of the mutans streptococci. Then, as the method for examining the caries risk capable of measuring the quantity of the mutans streptococci before infecting with said mutans streptococci, an attention have been attracted to the process, in which the antibody caused from a specific infection source exsisting in a human body is examined for judging whether a person is infected with a specific infection or not.

In a surface layer substance of the bacterial cell of the S. mutans being a kind of the mutans streptococci, it was identified that a protein antigen called as PAc (Protein Antigen cerotype C) with about 190000 molecular weight related with an initial adhesion of the mutans streptococci to a tooth surface in the research using the monoclonal antibody in which said protein antigen was used as the antigen. Moreover, a research for specifying a certain part in the PAc, which purely related with the initial adhesion of the mutans streptococci to the tooth surface, had been advanced, and it was identified that an A area having a *α* spiral structure in the PAc (a part of the amino acid sequence 216-464) strongly influenced colonization and adhesion on the tooth surface by the S. mutans, by Takahashi I. et al, "Immunogenicity and protective effect against oral colonization by Streptococcus mutans of synthetic peptides of a streptococcal surface protein antigen", J. Immunol, (USA), 1991, 146, p.332-6, by Takahashi I. Infect Immun (USA), Baltimore Md. American Association of Immunologists, 1992, 60, p.623-629, or by Okahashi N, et al, Mol. Microbiol. (USA), Blackwell Scientific Publications, 1993, 3, p.221-228. Furthermore, Senpuku being one of the present inventors et al. solved what was the most important sequence in the A area of the PAc (refer to nonpatent literature 1, Senpuku H, et al, "An antigenic peptide inducing cross-reacting antibodies inhibiting the interaction of Streptococcus mutans PAc with human salivary components" Infect Immun (USA), BaltimoreMd. American Association of Immunologists, 1995, 63, p.4695-4703). After that, it was identified that the sequence strongly acting on an human immune system as the antigen in the A area of the PAc was Y---L--Y, which was human B-cell epitope, and L--V-K--A, which was a part reacting with various human HLA-DR molecules, [refer to nonpatent literature 2, Senpuku et al, "Identification of Streptococcus mutans Pac peptide motif binding with human MHC class II molecules (DRBI * 0802, *1101, *1401, and *1405) Immunology (England), Blackwell Scientific Publications, 1998, 95, p.322-330, and nonpatent literature 3, Senpuku et al, "Inhibitory Effects of MoAbs against a Surface Protein Antigen in Real-Time Adherence In vitro and Recolonization In vivo of Streptococcus mutans" Scand. J. Immunol. (England), Oxford Blackwell Scientific Publications, 2001, 54, p. 109-116]. From these results, the specific amino acid sequence in the PAc, i.e., [NAKATYEAALKQYEADLAAVKKANAA (PAc(361-386))] was derived. The details of the amino acid sequence is indicated in the following formula.

As the result, the present inventors investigated that the examination of the caries risk could be accurately carried out by using the PAc (361-386), which is the amino acid sequence, as the antigen, and measuring an antibody value of a secretory immunoglobulin antibody A (sIgA) secreted from a human oral cavity mucosa, which was working to inhibit the adhesion of the mutans streptococci to the tooth surface.

However, as for measuring the antibody value of the secretory immunoglobulin antibody A against the PAc (361-386) secreted in the saliva, an infant of less than about 6 years old not having a completed immune function could not be examined, and since a saliva secretory quantity was varied with persons, the measured antibody value of the secretory immunoglobulin antibody A could not be accurately reflected between a person having a high quantity of the saliva and a person having a low quantity of the saliva. Furthermore, there were also problems that said measuring could not be applied to a patient infected with xerostomia, who has the remarkably low salivary secretion quantity, and an aged person weakened in his salivary secretion.

Then, the present invention has the object to provide the method for examining the caries risk capable of making all people into a sample irrespective of age and the salivary quantity.

The present inventors made wholeheartedly investigations in order to solve the above problems, and as the result, they paid attention to a major histocompatibility complex (hereinafter, it is sometimes referred to as only MHC) for examining the caries risk irrespective of the age and the salivary quantity. Although the MHC is found in cells of all higher vertebrates, since the MHC was first proved in leukocyte in the case of human, the MHC is called as a human histocompatibility leukocyte antigen or a human leukocyte antigen (hereinafter, it is referred to as HLA). It has been known that this HLA genes group has a remarkably important role in an immune system and functions as the antigen recognizing the non-itself.

Since a mechanism secreting the antibody against said PAc (361-386) naturally related to an immune system gene, the difference of the antibody value of the secretory immunoglobulin antibody A secreted in the saliva was determined with a specific genotype relating to the secretion of a human secretory immunoglobulin antibody A. Therefore, the present inventors investigated that it was possible to examine the caries risk by specifying an individual genotype, and if a human genotype was specified beforehand by high or low of the caries risk, that is, high or low of the antibody value of the secretory immunoglobulin antibody A secreted in the saliva, it was possible to carry out the method for examining the caries risk capable of targeting at all persons by only identifying the gene. Then, the present invention was completed.

The present invention is the method for examining the caries risk with the characteristics of identifying the genotype of DRB1* in the class II type of the HLA genes group, wherein the identified genotype is compared with the genotype specified for a caries risk, which is identified beforehand, derived from the antibody value (titer) of the secretory immunoglobulin A in the human saliva against antigen, in which a synthetic protein having an amino acid sequence composed of the following formula is used as the antigen.

In the HLA genes group, there are the class I type and the class II type, and these whole molecular structures closely resemble. In both of said classes, the antigen presented on a T cell bonds with an amino acid end domain, which has a membrane penetration type hetero dimer and comes out from the cells. The class II type exists in the limited cell, such as a B cell or an antigen presenting cell, and has the mechanism for promoting antibody production by the B cell thorough the T cell. In the present invention, the class II type gene group of the HLA genes group is especially used. As the class II type HLA, kinds of HLA-DR, DQ, DP and the likes have been known, and further, said HLA is composed of four domains of *α* 1, *α*2, *β*1 and *β*2. The HLA is specified by the HLA genes on sixth chromosome monobrachius, and has high polymorphism. Especially, it has been known that a *β* chain of the class II type DR gene (DRB1, DRB3, DRB4, DRB5) is further excellent in polymorphism, and recently, it has been known that these polymorphism genes are correlated with various diseases.

According to the method for examining the caries risk of the present invention, first DNA in cells collected from a patient is extracted to specify the HLA genes group on the sixth chromosome monobrachius. The caries risk is evaluated, by comparing the specified HLA genes group with the HLA genes group having high (or low) antibody value of the secretory immunoglobulin antibody A secreted in the saliva against the PAc (361-386), which is produced beforehand by the investigation.

Since the HLA genes group is existed in all cells of a person, the cell at any part of the person can be used as a sample. In said cells, for easily examining the caries risk in a dental clinic room, a human oral cavity mucosa cell, which is the easiest to be collected, is used preferably. As the method for collecting the oral cavity mucosa cell, the cells may be collected by scratching the mucosa with a spoon.

As the method for extracting the DNA from the collected cells, the general method for extracting the DNA from animal cells can be used. For example, a phenol chloroform method is preferable and various commercial kits can be used.

As the method for specifying the genotype, PCR-RFLP (polymerase chain reaction - restriction fragment length polymorphism), PCR-SSO (polymerase chain reaction - sequence specific oligonucleotide), PCR-SSP (polymerase chain reaction - sequence specific priming) or the likes can be used. However, as the method for specifying the gene, which is used in the method for examining the caries risk of the present invention, if there is any suitable method other than the above methods, it can be used.

The caries risk is evaluated, by comparing the specified HLA genes group with the HLA genes group having high (or low) antibody value of the secretory immunoglobulin antibody A secreted in the saliva against the PAc (361-386), which is produced beforehand by the investigation. For example, in the genotypes having been identified now, as the genotype having the high caries risk, that is, having the low antibody value of the secretory immunoglobulin antibody A, the case of the genotype having DRB1*0101, DRB1*0405, DRB1*0803, DRB1*1302, DRB1*1502 and DRB1*0410 at one side of the dimer, has been identified. Moreover, as the genotype having the low caries risk, that is, having the high antibody value of the secretory immunoglobulin antibody A, the case of the genotype having DRB1*0403, DRB1*0406, DRB1*0401, DRB1*0802, DRB1*0901, DRB1*1202, DRB1*1401, DRB1*1405, DRB1*1501 and DRB1*1602 at one side of the dimer, has been identified. In addition, if the combinations of all dimers are solved by the future research, the caries risk can be evaluated more accurately.

In order to obtain the PAc (361-386) being used in the present invention, although the method is not limited if the amino acid sequence of said PAc (361-386) can be obtained by said method, it is convenient to use an amino acid synthesizer in general.

As the method for measuring the antibody value of the secretory immunoglobulin antibody A being used in the method for examining the caries risk of the present invention, even if a conventional enzyme immunohistochemistry method, i.e., Enzyme Linked Immunosorbent Assay, which is referred to as "ELISA" hereinafter, being one of an antibody value measuring technique against the mutans streptococci, is used, sufficiently usable sensitivity can be obtained. However, the measurement sensitivity at the time of the determination can be increased by the technique being conventionally used for improving the sensitivity of the measured value obtained by the antigen antibody reaction, for example, the technique of reacting once the secretory immunoglobulin A in the saliva with a biotin anti-human immunoglobulin A etc. So, the technique based on the general antigen antibody reaction can be applied as it is. In addition to said technique, each technique of an immuno-chromatography, an immuno-concentration, and a latex agglutination etc. can be used suitably.

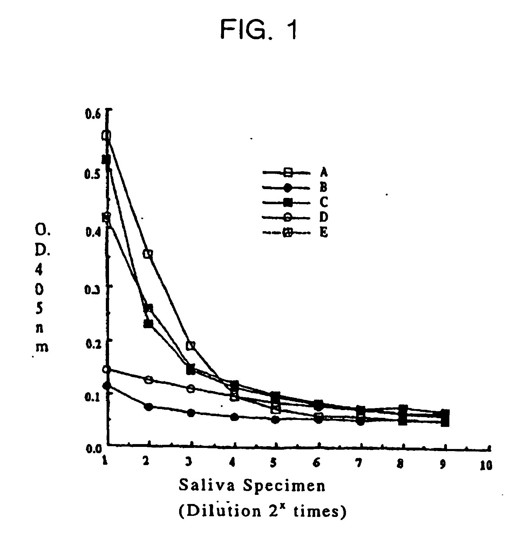

Figure 1 shows the results of measuring the antibody values of the immunoglobulin A in saliva of 5 subjects (A, B, C, D, E) against the PAc (361-386) with the ELISA.

Hereinafter, the method for examining the caries risk of the present invention will be explained concretely with the examples, but is not limited to the following examples. The operation was carried out at room temperature unless specifically described and pH is the value at 20 to 25 degree C. A concentration of protein quantity is calculated with absorbance of 280 nm wavelength light.

### Example 1

### <Method for Collecting Cells>

The oral cavity mucosa cell was collected, by rubbing a part of cheek in the oral cavity with the use of a swab (the product name was C. E. P. swab, produced by Lifetech Oriental Co. Ltd.). The extraction of the DNA was carried out immediately after the collection.

### <Extraction of the DNA from Oral Cavity Mucosa Cell>

The collected oral cavity mucosa cell was added to a 1.5 mL tube, which was added with 500 *µ*L of a DNAzol reagent (produced by Lifetech Oriental Co. Ltd.), and after homogenizing, was precipitated and washed with ethanol. Then, the DNA was extracted. The DNA concentration was identified with the absorbance of O. D. 260 nm wavelength light, and the extraction purity was identified with the absorbance of O. D. 260 nm / O. D. 280 nm wavelength light. The extracted DNA was suspended in TE buffer, comprising tris (hydroxymethyl) aminomethane 10 mM, ethylene diamine tetraacetic acid (EDTA) 1 mM with pH 8.0. Then the following operation was carried out.

### <Determination of Genotype of DRB1*>

The determination of the genotype of DRB1* was carried out by using the following PCR-RFLP, in accordance with "PCR-RFLP typing detects new HLA-DRB1 alleles : DRB1*13022, DRB1*1336 and DRB1*1435" (European Journal of Immunogenetics, 28, 441-447) by Trejaut J. et al. The conditions of the PCR were as follows.

The above-described purified DNA: 100 to 500 ng

| | |
|---|---|
| MgCl₂ | : 1.5 mM |
| dNTPs | : 40 *µ* M |
| Dimethyl sulfoxide (DMSO) | : 3 % by weight |
| The following primer | : 50 pM |

AmpliTaq (PE Biosystems, Foster City, CA, USA): 1 unit
All of above was mixed and a total of 50 *µ*L was prepared.

As the primer,
DR3 (5' cacgtttcttggagtactc 3') (Horne & Keown, 1993) and
AmpB (5' ccgctgcactgtgaagctct 3') (Kimura & Sasazuki, 1992)
were used to DRB1*03, 08, 11, 12,13,14. After amplification, an amplified fragment of 266 bp was obtained from exons 2. In addition, as for DRB1*1122. 1410, DR4-like was used instead of the primer DR3 in 5'-side, and as for DRB1*1130, DR4-like was also used. (refer to Marsh, S. G. E., HLA class II region sequence 1998, Tissue Antigen : 51, 467-507.)

As for the prepared sample, the specific gene sequence was amplified, by using a thermal cycler (the product name was GeneAmp PCR System 9700 Thermal Cycler, produced by PE Biosystems Company). As for the operations of the thermal cycler program, the operations were carried out at 95 degree C for 2 minutes, at 94 degree C for 30 seconds, at 60 degree C for 30 seconds, and at 72 degree C for 30 seconds and these operations were repeated 30 times. After that, the operation was carried out at 72 degree C for 10 minutes. As for identifying the amplification sequence, 5 *µ*L of the above-described PCR product was electrophoresed in an agarose gel, which was 2% of 1 × TAE (tris (hydroxymethyl) aminomethane was 0.04 M, acetate was 0.04 M, EDTA was 0.001 M) containing 0.2 *µ*g / mL of ethylene bromide, by using an electrophoresis device (the product name was Mupid 2, produced by Cosmobio Co. Ltd.). As for the electrophorsed product, an amplification product was identified by using a UV - Transilluminator (produced by Funakoshi Co. Ltd.).

### < Restriction Enzyme Treatment >

After the electrophoresis, restriction enzyme treatment was carried out. That is, BseRI (4 Unit), BsaJI (3 Unit), RsaI (5 Unit) and Sau96I (5 Unit), (all produced by New England Biolabs Company), were used. As for the restriction enzyme treatment, the treatment was carried out at 37 degree C for one night, by using said enzyme and refined water, in which a 1 × buffer solution (provided together with the enzyme) was used to 10 *µ* L of the PCR product. However, as for only the BsaJI, the treatment was carried out at 60 degree C for one night. The restriction treated sample was electrophorsed with the conditions of 500 V for one hour, by using 10 % polyacrylamide gel, which is prepared with 1 × TBE (tris (hydroxymethyl) aminomethane was 0.089 M, boron was 0.089 M, EDTA was 0.02 M). As for the electrophoresis, a 100bpDNA (produced by Takara Shuzo Co. Ltd.) was used as a size marker by feeding to a 1 lane and the electrophorsed product was identified. After the electrophoresis, the gel was removed from a glass board, and immersed in and dyed with the solution, in which 0.8 *µ*g./ mL ethidium bromide was dissolved with 1 × TBE. The identification of the gel was carried out by taking a photograph using the UV - Transilluminator (produced by Funakoshi Co. Ltd.) and a gel movie camera (the product name was Funaroid Camera FR6000, produced by Funakoshi Co. Ltd.).

### <Determination of DRB1 Sequence>

The genotype of DRB1 was determined in accordance with the above-described method.

### <Measurement of Saliva Secretory Antibody with ELISA>

### (1) Synthesis of PAc (361-386)

The peptide having the amino acid sequence of PAc (361-386) was made by a stepwise solid-phase peptide synthesis method. Model 350 Multiple Peptide Synthesizer (the product name was Advanced Chemitech, produced by Louisville Company), was used as a synthesizer. The examination of the synthetic peptide was carried out by the high-performance reverse phase liquid chromatograph using TSK-GEL column (30×1), i.e., 10-45 % acetonitrile was used as a column and gradient was 0.1 % TFA. The last refining degree was more than 95 %.

### (2) Measurement of Saliva Secretory Antibody

### A) Forming into solid phase of the antibody

The PAc (361-386) synthesized by the above process was diluted with a 50mM sodium carbonate buffered solution having pH of 9.6, and this diluted PAc (361-386) was added to each well of a 96 hole microplate (produced by Sumitomo Bakelite Company) so as to become 100 *µ*L. The added PAc (361-386) was kept quietly overnight at 4 degree C to bind with the well.

### B) Blocking

The microplate with the solid phase being formed by the above process was washed three times with a liquid (referred to as PBST hereinafter), in which 0.05 % by weight neutral interfacial active agent (the product name was TWEEN20 produced by SIGMA Company) was contained in a PBS (Phosphate Buffered Saline having pH of 7.4), by using a microplate washer (the product name was Model 1575 produced by Bio-Rat Company). After the washing, the PBS containing the 1 % skim milk was injected by 200 *µ*L per each well, and blocking was carried out at 37 degree C for one hour. Af ter the blocking, washing was carried out three times by the PBST to wash an excessive blocking agent.

### C) Preparation of Saliva

The stimulus saliva secreted by biting a paraffin wax for 3 minutes was used as the specimen. After obtaining, the salivary specimen was preserved in ice until starting the measurement. In the experiment, the saliva was prepared by diluting 2, 4, 8, 16, 32, 64, 128, 256, 512, and 1024 times with the PBST containing 0.5 % skim milk. Each of the saliva was added by 100 *µ*L to said plate. After adding, the plate was kept quietly at 37 degree C for one hour and washed with the PBST.

### D) Labeled Antibody

The anti-human IgA antibody labeled with an alkaline phosphatase (the product name was Anti-Human-IgA, produced by Chemicon International Company), was prepared with the PBST containing 0.5 % by weight of skim milk so as to be 0.3 *µ*g / mL. 100 *µ*L of the prepared label was added to the each well and reacted at 37 degree C for one hour.

### E) Coloring

After washing with the PBST, 100 *µ*m of a diethanolamine buffer containing 0.1 % by weight of P-nitrophenol disodium phosphate hexahydrate, which is a coloring substrate of alkaline phosphatase was added to each well and kept quietly at 37 degree C for 30 minutes. Then, the absorbance of 405 nm wavelength light was measured. These results were shown in Table 1. In this case, the diethanolamine buffer was prepared with the following prescription.
Diethanolamine : 48.5 mL,
MgCl₂·6H₂O : 50.0 mg,
Na₃N : 100 mg,
H₂O : 400 mL,
After preparing the final pH to 9.8, water was added to the diethanolamine buffer to be 500 mL.

The antibody values of the immunoglobulin A in the saliva of five subjects (A, B, C, D, E) against the PAc (361-386) were measured with the ELISA, and these results were shown in Figure 1. As the result, grouping was made into two, i.e., the high antibody value group (in this case, A, C, E) and the low antibody value group (in this case, B, D). In addition, the accuracy of the method for examining the caries risk was identified between the actual quantity of the mutans streptococcus in the oral cavity and the groups of the antibody value being high and low.

The genotypes of HLA-DRB1* of each person were identified with the oral cavity mucosa cells of the above subjects (A, B, C, D, E) by the above process, and the correlation between the genotypes and the antibody value of the secretory immunoglobulin A in the saliva against the PAc (361-386) was identified. These results were shown Table 1. By setting such condition, the genotypes respectively corresponding to the above two groups, i.e., the high antibody value group (A, C, E) and the low antibody value group (B, D), was identified beforehand by using a plurality of subjects as the sample. In addition, it was identified that the genotype of HLA-DRB1* was correlated with the antibody value.

**[Table 1]**

| Antibody Value (O.D. 405 nm) | Subjects Subjects | Genotype of DRB1* in Class II Type of HLA gene Group |
|---|---|---|
| High | A | 0403, 0405 |
| | C | 1501, 0410 |
| | E | 1602, 1405 |
| Low | B | 0101, 1502 |
| | D | 0803, 0803 |

As for the actual method for examining the caries risk, when the above-described PAc (361-386) being identified beforehand is used as the antigen, the caries risk is examined, by comparing the genotype relating to high or low of the caries risk derived from high or low of the antibody value of the secretory immunoglobulin A in the human saliva against the antigen, with the genotype identified with the subject.

As described above, the method for examining the caries risk of the present invention is the method capable of subjecting all people to examination irrespective of the age and the salivary quantity as compared with a conventional immunity method. In addition, since caries risk can be more accurately and widely examined by the present invention, this method has the great value for contributing to dental medicine.

## Claims

1. A method for examining a caries risk **characterized by** identifying a genotype of DRB1* in a class II type of a HLA gene group, wherein the identified genotype is compared with the genotype specified for a caries risk; which is identified beforehand, derived from an antibody titer of a secretory immunoglobulin A in human saliva against an antigen, in which a synthetic protein having an amino acid sequence composed of the following formula is used as the antigen.

## Patentansprüche

1. Verfahren zum Untersuchen eines Kariesrisikos, **gekennzeichnet durch** das Identifizieren eines Genotyps von DRB1* in einem Klasse-II-Typ einer HLA-Gengruppe, wobei der identifizierte Genotyp verglichen wird mit einem für ein Kariesrisiko spezifierten Genotyp, der zuvor identifiziert wird, abgeleitet von einem Antikörpertiter eines sekretorischen Immunglobulins A in menschlichem Speichel gegen ein Antigen, in dem ein synthetisches Protein mit einer Aminosäuresequenz, zusammengesetzt aus der folgenden Formel, als das Antigen verwendet wird: 1

## Revendications

1. Procédé pour évaluer un risque de carie **caractérisé par** l'identification d'un génotype de DRB1* dans un type de classe II d'un groupe de gènes HLA,
dans lequel le génotype identifié est comparé au génotype spécifié pour un risque de carie, qui est identifié au préalable, dérivé d'un titre d'anticorps d'une immunoglobuline A sécrétoire dans la salive humaine contre un antigène, dans lequel une protéine synthétique ayant une séquence d'acides aminés composée de la formule suivante est utilisée en tant qu'antigène.


### SEQUENCE LISTING

<110> GC Corporation
<120> Method for examining the caries risk
<130> G 4268
<140> EP 03026551.6
   <141> 2003-11-18
<150> Japanese Patent Application No. 2002-352466
   <151> 2002-12-04
<160> 3
<170> Patent In version 3.1
<210> 1
   <211> 26
   <212> PRT
   <213> artificial sequence
<220>
   <223> synthetic protein
<400> 1
   Asn Ala Lys Ala Thr Tyr Glu Ala Ala Leu Lys Gln Tyr Glu Ala Asp
   1 5 10 15
   Leu Ala Ala Val Lys Lys Ala Asn Ala Ala
   20 25
<210> 2
   <211> 19
   <212> DNA
   <213> artificial sequence
<220>
   <223> primer for determination of the genotype of DRB1*
<400> 2
   cacgtttctt ggagtactc
   19
<210> 3
   <211> 20
   <212> DNA
   <213> artificial sequence
<220>
   <223> primer for determination of the genotype of DRB1*
<400> 3
   ccgctgcact gtgaagctct
   20